# EUROPEAN PATENT SPECIFICATION

(11) **EP 1 803 483 B1**
(45) Date of publication and mention of the grant of the patent: **25.05.2011**
(21) Application number: 06126691.2
(22) Date of filing: 20.12.2006
(51) Int. Cl.: A61M 25/09, C21D 1/09

(54) **Guide wire**
Führungsdraht
Fil de guidage

(30) Priority: 27.12.2005 JP 2005376710; 14.11.2006 JP 2006307940
(43) Date of publication of application: 04.07.2007
(73) Proprietor: Terumo Kabushiki Kaisha, Tokyo (JP)
(72) Inventor: SOUBA, Ryouichi, Fujinomiya-shi Shizuoka (JP); MURAYAMA, Hiraku, Fujinomiya-shi Shizuoka (JP)
(74) Representative: TBK

(56) References cited:
- GB-A- 2 160 227
- GB-A- 2 245 680
- US-A- 4 304 978
- US-A1- 2004 220 499
- US-A1- 2005 049 690
- US-A1- 2005 182 478
- P. PEYRE, X. SCHERPEREEL, L. BERTHE, C. CARBONI, R. FABBRO, G. BÉRANGER, C. LEMAITRE: "Surface modifications induced in 316L steel by laser peening and shot-peening. Influence on pitting corrosion resistance" MATERIALS SCIENCE AND ENGINEERING A, vol. 280, no. 2, 31 March 2000 (2000-03-31), pages 294-302, XP002428758

## Description

### BACKGROUND OF THE INVENTION

### 1. Field of the Invention

The present invention relates to a guide wire and, more particularly, to a guide wire that is used to introduce a catheter into a body cavity, specifically blood vessel and bile duct.

### 2. Description of the Related Art

A guide wire is a device designed to guide a catheter for examination (such as cardioangiography) and also for therapy of sites involving difficulties in surgical operation such as percutaneous transluminal coronary angioplasty (PTCA) and for low-invasive therapy. PTCA requires a guide wire to reach the vicinity of the narrow segment of the coronary artery, thereby guiding the forward end of a balloon catheter to the desired position. In this case, a guide wire is used in such a way that its forward end projects from the forward end of a balloon catheter.

A guide wire is also used to guide a balloon catheter to the narrow segment for percutaneous transluminal angioplasty (PTA) in the same way as for PTCA. In this case, the object of a balloon, catheter is to reopen the narrow or occluded segment in peripheral vessels of femoral, iliac, renal, and shunt.

Another use of a guide wire is for the therapy of bile duct and pancreatic duct as exemplified below, in which a guide wire brings a therapeutic device to a lesion.
(1) Endoscopic retrograde cholangiopancreatography (ERCP)
   This operation involves insertion of an endoscope into the descending part of the duodenum, insertion of a cannula into the bile duct or pancreatic duct with the Vater papilla visible in the front of the endoscope, and injection of a contrast medium for X-ray photography.
(2) Endoscopic sphincterotomy (EST)
   This operation involves insertion of a papillotome into the opening of the duodenum papilla and incision of the papillary sphincter by high frequency.
(3) Endoscopic papillary balloon dilation (EPBD)

This operation involves expansion of a papilla by a balloon through an endoscope and elimination of biliary gallstones.

The fact that the blood vessel requiring PTCA curves intricately calls for a guide wire to assist insertion of a balloon catheter into the vessel which has good steerability and good kink (or bend) resistance. Steerability includes flexibility that permits adequate bending, ability for restoration, pushability for smooth insertion, and torque transmittability for conveyance of manipulation at the base end to the forward end.

There are some known guide wires to meet these requirements. The first one has adequate flexibility owing to a flexible bendable metal coil formed around the core wire at its forward end. The second one has adequate flexibility and restorability owing to the core wire made of superelastic alloy. The third one has improved torque transmittability owing to the core wire made of stainless steel.

On the other hand, there is a growing demand for the guide wire with a smaller diameter as the catheter (for insertion into the living body) is made thinner in order to reduce loads on patients.

Unfortunately, the conventional guide wire mentioned above involves difficulties in further reduction of its diameter because of its intrinsic structure, or it can be made thin only with the sacrifice of bending and torsional stiffness and steerability such as pushability and torque transmittability. The resulting thin guide wire would be unable to guide the balloon catheter smoothly and adequately to the desired position.

Document US 2005/0182478 A1 discloses a laser shock peening process for producing one or more compressive residual stress regions in a medical device. A surface of a workpiece is hardened by exposing it to an intense laser beam. Thereby, a pattern of hardened surface areas is produced in the workpiece.

Document US 2004/0220499 A1 discloses a medical instrument according to the preamble of claim 1. The medical instrument mainly consists of a guidewire consisting of alternately arranged untreated and annealed sections. The material for the guidewire is stainless steel.

Document GB 2 160 227 A discloses a heat treatment process for transformation hardening which provides a line heat-input pattern which is scanned along the surface of a treated material.

It is the object of the invention to provide a guide wire having an increasing flexural and torsional stiffness at one of its ends to improve steerability and kink resistance.

The object of the invention is achieved by a guide wire according to claim 1. Advantageous embodiments are carried out according to the dependent claims.

A guide wire provides a wire member having a flexible core wire and a hardened part formed at least on the surface thereof by hardening treatment. In the guide wire, the hardened part has an odd-shaped part in a specific region along the lengthwise direction of the core wire, the odd-shaped part assuming a straight pattern, curved pattern, annular pattern, spiral pattern, or reticulate pattern, or a combination of two or more of them.

The hardened part preferably has an average Vickers hardness of 1.02 K to 3.5 K, where K denotes a Vickers hardness at that part of the core wire which has not yet undergone hardening treatment.

The guide wire preferably has a part in which the hardened parts in the odd-shaped part are formed at intervals decreasing in going toward one end of the core wire.

The guide wire preferably has a part in which the hardened part in the periphery of the core wire assumes a stripe pattern changing in width along the lengthwise direction of the core wire.

The guide wire preferably has the hardened part such that at least part of it is near or at the center of the core wire.

The guide wire preferably has a part in which the total sectional area of the hardened part is smaller than the total sectional area of the unhardened part without hardening treatment of the core wire.

The guide wire preferably has a part in which the total sectional area of the hardened part is equal to or larger than the total sectional area of the unhardened part without hardening treatment of the core wire.

The guide wire preferably has a part in which the total sectional area of the hardened part varies in the lengthwise direction of the core wire.

The core wire is preferably formed from a ferroalloy.

The ferroalloy is preferably at least one species selected from the group including stainless steel, piano wire, iron-cobalt alloy, any other cobalt alloys, carbon steel, mild steel, hard steel, nickel steel, nickel-chrome steel, and nickel-chrome-molybdenum steel.

The core wire is preferably made of a metallic material containing no less than 0.1 wt% of carbon.

The hardened part preferably has a part varying in hardness in the radial direction and/or the lengthwise direction of the wire member.

The hardened part is preferably formed by heating and quenching.

The heating is preferably accomplished by irradiation with a laser beam.

The hardened part is preferably formed next to the odd-shaped part and has an approximately straight part arranged in the lengthwise direction of the core wire.

The hardened part is preferably arranged in the odd-shaped part more densely in the base end side of the wire member than in the forward end side of the wire member.

The guide wire preferably has a covering layer that covers the wire member.

The guide wire according to the present invention is characterized by having the hardened part which makes its specific part (such as the base end) sufficiently stiff even when it has a reduced diameter, not to mention when it has an ordinary diameter. Consequently, it is superior in steerability such as pushability and torque transmittability and kink resistance. It possesses sufficient stiffness without sacrificing flexibility at its forward end. The improved torque transmittability is due to the odd-shaped part in the hardened part.

The guide wire according to the present invention varies from part to part in physical properties because the hardened part, particularly the odd-shaped part, of the wire member varies from part to part (say, base end, intermediate part, and forward end) in shape, arrangement, sectional area, hardening depth, arrangement density, and hardness as desired. Consequently, it can be properly applied to any lesion and case. It has good pushability, torque transmittability, and kink resistance when it is constructed such that it is sufficiently flexible at its forward end and gradually increases in stiffness in going toward the base end.

The guide wire according to the present invention has the hardened part which is formed by hardening treatment on the core wire of the wire member; therefore, the hardened part remains in the wire member and takes on varied patterns.

The hardened part may have an intricate or minute pattern when it is formed by irradiation with a laser beam, followed by quenching. In this case, the pattern of the hardened part on the outside of the wire member corresponds to the pattern of laser beam irradiation. An adequate selection of the laser beam energy makes it possible to control the depth of the hardened part and the ratio of the sectional area of the hardened part in the wire member.

The guide wire according to the present invention easily slides in a catheter for good steerability if it is provided with a covering layer, particularly one which is made of a plastic material that reduces friction. The reduced sliding resistance certainly prevents kinking and twisting and improves steerability.

### BRIEF DESCRIPTION OF THE DRAWINGS

Fig. 1 is a partly sectional view showing a guide wire according to one embodiment of the present invention;
Fig. 2 is a perspective view showing the structure of the wire member in the region [A] of the guide wire shown in Fig. 1;
Fig. 3 is a perspective view showing the structure of the wire member in the region [A] of the guide wire shown in Fig. 1;
Fig. 4 is a perspective view showing the structure of the wire member in the region [A] of the guide wire shown in Fig. 1;
Fig. 5 is a perspective view showing the structure of the wire member in the region [A] of the guide wire shown in Fig. 1;
Fig. 6 is a perspective view showing the structure of the wire member in the region [A] of the guide wire shown in Fig. 1;
Fig. 7 is a perspective view showing the structure of the wire member in the region [A] of the guide wire shown in Fig. 1;
Fig. 8 is a perspective view showing the structure of the wire member in the region [A] of the guide wire shown in Fig. 1;
Fig. 9 is a perspective view showing the structure of the wire member in the region [A] of the guide wire shown in Fig. 1;
Fig. 10 is a perspective view showing the structure of the wire member in the region [A] of the guide wire shown in Fig. 1;
Fig. 11 is a perspective view showing the structure of the wire member in the region [B] of the guide wire shown in Fig. 1;
Fig. 12 is a sectional view showing the structure of the wire member in the region [A] of the guide wire shown in Fig. 1;
Fig. 13 is a sectional view showing the structure of the wire member in the region [A] of the guide wire shown in Fig. 1;
Fig. 14 is a sectional view showing the structure of the wire member in the region [A] of the guide wire shown in Fig. 1;
Fig. 15 is a sectional view showing the structure of the wire member in the region [A] of the guide wire shown in Fig. 1;
Fig. 16 is a schematic diagram illustrating how to use the guide wire according to the present invention;
Fig. 17 is a schematic diagram illustrating how to use the guide wire according to the present invention;
Fig. 18 is a diagram illustrating how to test the guide wire according to the present invention for torque transmittability; and
Figs. 19A and 19B are graphs showing the results of test for torque transmittability performed on the guide wire according to the present invention. The results are expressed in terms of time vs. angle of rotation.

### DETAILED DESCRIPTION OF THE PREFERRED EMBODIMENT

A detailed description is given below of the guide wire according to a preferred embodiment of the present invention as illustrated in the accompanying drawings.

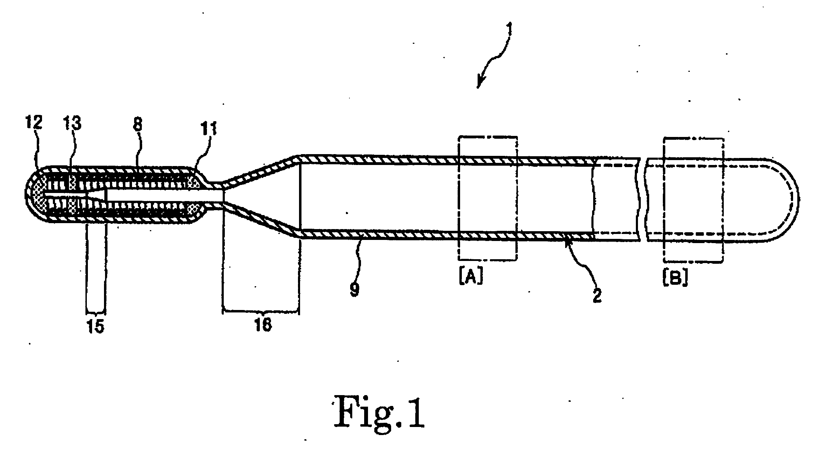

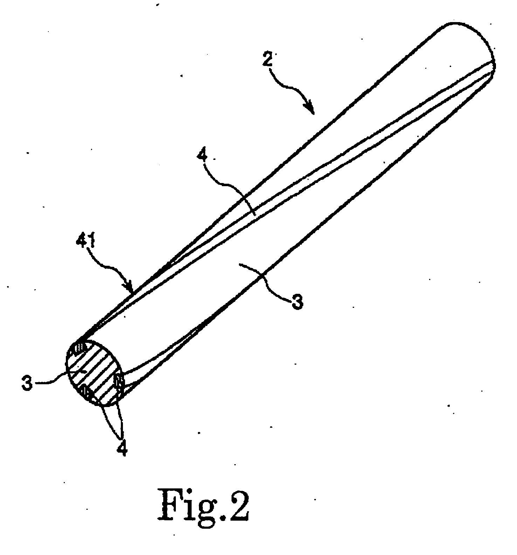

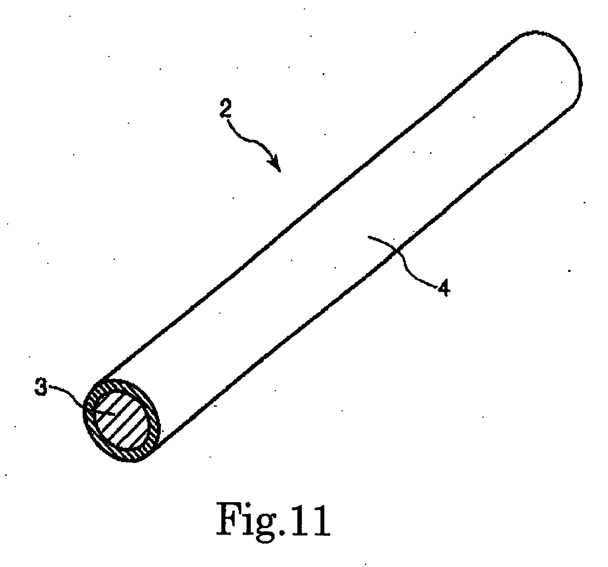

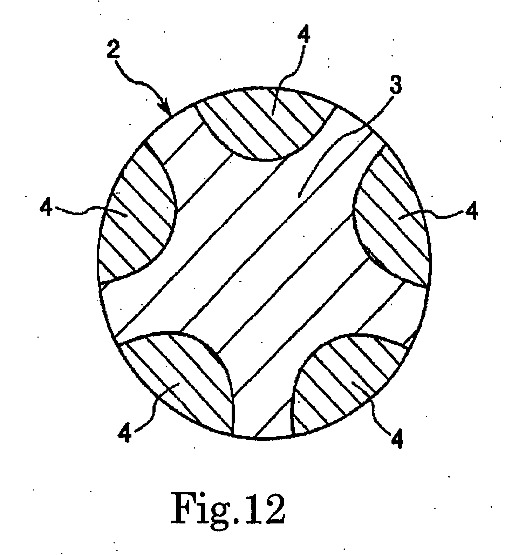

Fig. 1 is a partly sectional view showing a guide wire according to one embodiment of the present invention. Figs. 2 to 10 are perspective views each showing the structure of the wire member in the region [A] of the guide wire shown in Fig. 1. Fig. 11 is a perspective view showing the structure of the wire member in the region [B] of the guide wire shown in Fig. 1. Figs. 12 to 15 are sectional views each showing the structure of the wire member (core wire) in the region [A] of the guide wire shown in Fig. 1.

For the sake of brevity in the following description, the term "base end" is used to denote the right side in Fig. 1 and the upper right side in Figs. 2 to 11, and the term "forward end" is used to denote the left side in Fig. 1 and the lower left side in Fig. 2 to 11. Fig. 1 is a schematic diagram to help understanding in which the guide wire is contracted in its lengthwise direction and exaggerated in its sectional direction, with the scales in both directions differing. Incidentally, Fig. 1 does not show the hardened part 4.

The guide wire 1 shown in Fig. 1 is one which is used to guide a catheter (and endoscope) for insertion into a body cavity. It has as the major constituent the wire member 2 (with a circular cross section) composed of one or more flexible core wires.

The illustrated wire member 2 is constructed of one continuous core wire; however, the present invention covers the one which is constructed of two or more core wires (welded together) of different or identical material.

The guide wire 1 should preferably have an overall length of 200 to 5,000 mm, which is not specifically restricted.

According to the illustrated embodiment, the wire member 2 is composed of two parts -- one with a constant diameter and one with a gradually decreasing diameter (which is referred to as a tapering part). The wire member 2 may have one or more tapering parts, like the one shown in Fig. 1 which has two tapering parts 15 and 16.

The tapering parts 15 and 16 make the wire member (core wire) 2 gradually decrease in bending and torsional stiffness in going to its forward end. The resulting guide wire 1 has an adequately flexible forward end, so that it easily follows the blood vessel safely without bending.

The illustrated structure having the tapering parts 15 and 16 formed partially in the lengthwise direction of the wire member 2 may be modified such that the wire member 2 as a whole is gradually tapered. The tapering parts 15 and 16 may have a taper angle (or the rate of decrease in diameter) which remains constant or changes in the lengthwise direction of the wire member 2. For example, there may be tapering parts with a comparatively large taper angle and a comparatively small taper angle which are arranged alternately.

The wire member 2 has a constant diameter over its length from the tapering part 16 to the base end.

The core wire constituting the wire member 2 has the hardened part 4 in a specific pattern, which is formed by hardening treatment on at least the surface thereof. The other part than the hardened part 4 is the unhardened part 3.

The hardened part 4 imparts desired bending and torsional stiffness to the wire member 2 in addition to the intrinsic properties determined by selection of material and diameter. The resulting guide wire 1 has desired flexibility as well as improved steerability and kink resistance.

The hardened part 4 is not formed by providing the core wire of the wire member 2 with an additional material by welding or embedding but is formed by modifying the constituent material of the core wire by subjecting the core wire of the wire member 2 to hardening treatment such as heating and quenching. The hardened part 4 formed in this manner never peels off from the wire member 2 and it takes on any desired pattern.

The hardened part 4 is harder than the unhardened part 3, and the difference in hardness between them is defined as follows. That is, the hardened part 4 should have an average Vickers hardness (Hv) of 1.02 K to 3.5 K, preferably 1.05 K to 2.0 K, where K denotes a Vickers hardness at the unhardened part 3. If this value is smaller than specified, the hardened part 4 will not fully function. The greater difference in hardness than specified above will not be realized by the hardening method employed.

The hardened part 4 may have a hardness which is uniform throughout it or which is varied from position to position. For example, the harness may vary in the lengthwise direction or the radial direction. In this case, an average value should be obtained from measurements at different positions.

The hardened part 4 may vary in desirable Vickers hardness (Hv) depending on the material constituting the wire member 2. If the core wire of the wire member 2 is made of stainless steel (SUS) or nickel-chrome-molybdenum steel, the hardened part 4 should have an average Vickers hardness of about 300 to 800 or about 300 to 900, respectively. The hardness of the hardened part 4 may be properly controlled by selection of material and quenching rate in the hardening treatment.

The hardened part 4 may be formed in any manner without specific restrictions. A preferred method is heating (by irradiation with a laser beam) and ensuing quenching. This method is suitable for the hardened part 4 with a complicated shape and a fine pattern. Irradiation with a laser beam forms the hardened part having the same pattern as the irradiation pattern on the surface of the wire member 2. Moreover, the hardened part 4 varies in depth (in the radial direction of the wire member 2) depending on the energy of the laser beam for irradiation (which is determined by power of laser source, condensed density, temperature, and duration of irradiation). The hardened part 4 will reach the center of the wire member 2 if the laser beam for irradiation has a sufficient energy.

The laser for irradiation includes, for example, carbon dioxide gas laser, YAG laser, excimer laser, Ar laser, He-Ne laser, semiconductor laser, glass laser, and ruby laser.

Heating by irradiation with a laser beam should be followed by quenching at a rate of about 1 to 2, 000°C/s, preferably 800 to 1,500°C/s. The greater the quenching rate, the higher the hardness of the hardened part 4.

Additional heating methods include plasma irradiation, discharging, high-frequency induction heating, microwave heating, and millimeter wave heating.

The wire member 2 may be formed from any material without specific restrictions. Typical examples include stainless steel (such as SUS304, SUS303, SUS302, SUS316, SUS316L, SUS316Jl, SUS316J1L, SUS405, SUS430, SUS434, SUS444 , SUS429 , SUS430F, and SUS302), piano wire, ferroalloys (such as iron-cobalt alloy, carbon steel such as extremely low carbon steel and low carbon steel, mild steel, hard steel, nickel steel, nickel-chrome steel, and nickel-chrome-molybdenum steel), cobalt alloy, titanium alloy, and nickel alloy.

A preferred material for the core wire is a ferroalloy containing equal to or more than 0.1 wt% (preferably 0.1 to 2 wt%) of carbon (C). Such a ferroalloy is exemplified by a carbon steel containing 0.3 to 2 wt% of carbon. A eutectoid carbon steel is preferable for the following reason.

When the hardened part 4 is formed by heating (by irradiation with a laser beam) and ensuing quenching as mentioned above, the constituent material of the core wire undergoes transformation from austenite into martensite, pearlite, and bainite, thereby increasing in hardness. Since transformation is due to carbon (C), a eutectoid carbon steel capable of eutectoid transformation is desirable.

Another preferred material for the core wire is nickel-chrome-molybdenum steel, such as those alloys composed of 0.4 to 3.5 wt% Ni, 0.4 to 3.5 wt% Cr, 0.15 to 0.7 wt% Mo, 0.2 to 0.5 wt% C, and Fe (remainder), with other optional elements such as Ti, Nb, Si, Mn, P, S, Cu, and V.

Nickel-chrome-molybdenum steel is preferable because it readily hardens upon heating or it readily gives the hardened part 4 in widely varied patterns. The above-mentioned optional elements promote transformation into pearlite and bainite.

The hardened part 4 should be different in metallographic structure from the unhardened part 3 (the rest of the core wire of the wire member 2). In other words, the unhardened part 3 should have the structure of austenite and the hardened part 4 should have any one structure of martensite, pearlite, and bainite. The wire member 2 composed of the hardened part 4 and the unhardened part 3, which differ in metallographic structure, partially varies in stiffness, so that it exhibits good pushability and torque transmittability despite its small diameter.

The hardened part 4 may have one or more than one metallographic structure of martensite, pearlite, and bainite. With more than one metallographic structure varying in hardness, the hardened part 4 may have a desirable hardness according to the content of the individual structures.

The hardened part 4 has the odd-shaped part 41 in a specific region in the lengthwise direction of the wire member (core wire) 2. This region in indicated by [A] in Fig. 1 illustrating the embodiment. The odd-shaped part 41 has a straight, curved, circular, spiral, or reticulate pattern, or a combination thereof.

The following is a description of the pattern possessed by the hardened part 4, especially the odd-shaped part 41. In the example shown in Fig. 2, the wire member (core wire) 2 has the hardened parts 4 (three in total) formed in the periphery thereof. The hardened parts 4 include spirally elongated patterns arranged at equal intervals in the circumferential direction.

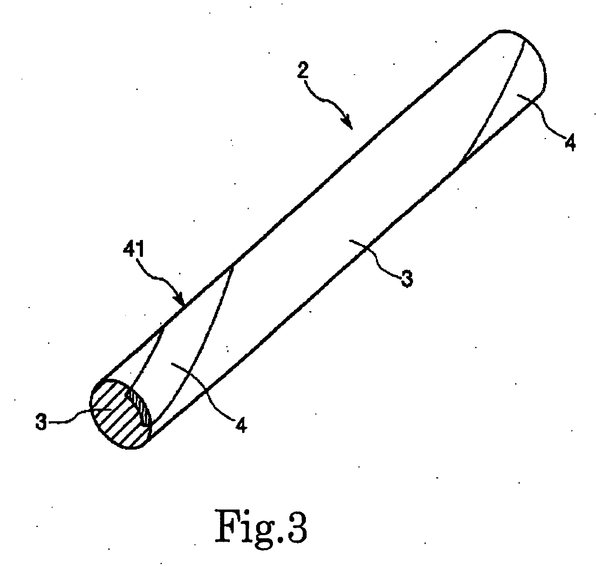

In the example shown in Fig. 3, the wire member (core wire) 2 has the hardened part 4 (in one spiral pattern) formed in the periphery thereof.

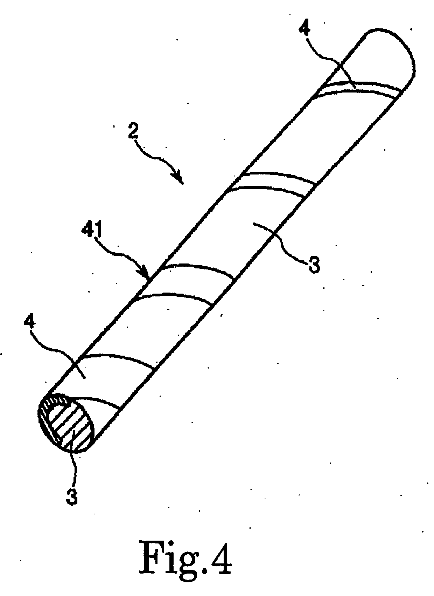

In the embodiment shown in Fig. 4, the wire member (core wire) 2 has the hardened part 4 (with a spiral stripe pattern) formed in the periphery thereof. The stripe pattern gradually increases in width in going to the forward end. In other words, the stripe pattern is formed such that the hardened part 4 accounts for a larger portion in the core wire in going to the forward end. The hardened part 4 formed in this manner makes the wire member (core wire) 2 gradually increase in flexural and torsional stiffness in going from the base end to the forward end.

The number of the hardened parts 4 may be two or more as shown in Fig. 2. Moreover, it may increase or decrease as they branch out. The hardened part 4 may be formed such that its width gradually increases in going from the forward end to the base end.

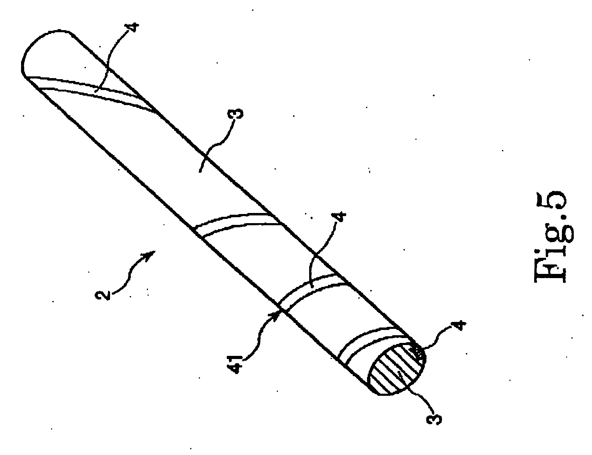

In the embodiment shown in Fig. 5, the wire member (core wire) 2 has the hardened part 4 with a spiral stripe pattern formed in the periphery thereof. The stripe pattern gradually decreases in pitch (interval) in going to the forward end. In other words, the stripe pattern is formed such that the hardened part 4 accounts for a larger portion in the core wire in going to the forward end. The hardened part 4 formed in this manner makes the wire member (core wire) 2 gradually increase in flexural and torsional stiffness in going from the base end to the forward end.

The number of the hardened parts 4 may be two or more as shown in Fig. 2. Moreover, it may increase or decrease as they branch out. The hardened part 4 may be formed such that its spiral pitch gradually decreases in going from the forward end to the base end.

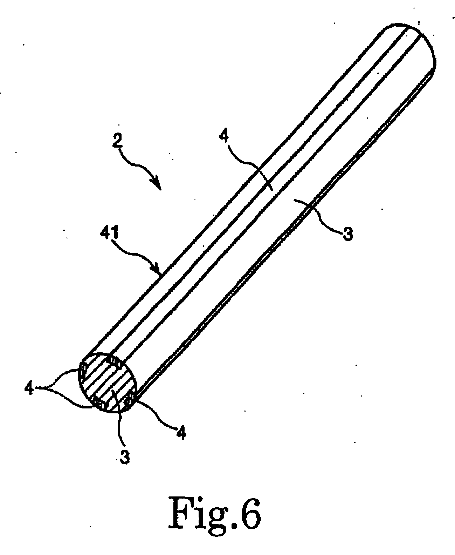

In the example shown in Fig. 6, the wire member (core wire) 2 has the straight hardened part 4 (four in total, extending in the lengthwise direction) formed in the periphery thereof. Each of the hardened part 4 should be arranged at equal intervals in the circumferential direction.

In the example shown in Fig. 6, each of the straight hardened part 4 has an equal width. However, the hardened part 4 may be formed such that its width gradually increases or decreases in going from the base end to the forward end. The hardened part 4 formed in this way makes the wire member (core wire) 2 gradually increase or decrease in flexural and torsional stiffness in going from the base end to the forward end. The number of the hardened parts 4 may increase or decrease as they branch out without altering the above-mentioned effect.

The hardened part 41 shown in Fig. 6 may be formed in such a pattern that the adjoining linear parts are connected to each other (not shown) by circular, spiral, or reticulate parts.

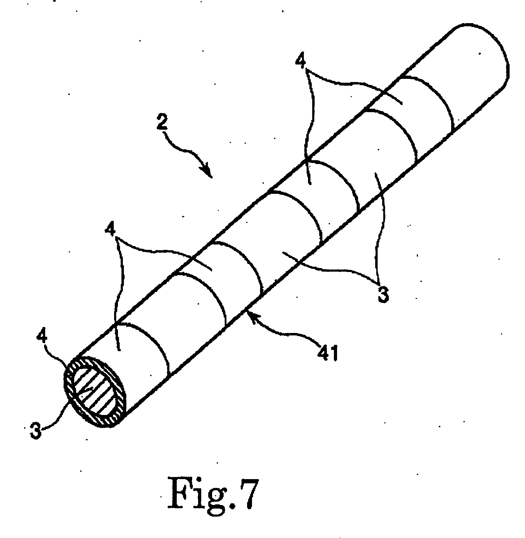

In the embodiment shown in Fig. 7, the wire member (core wire) 2 has the hardened parts 4 (counting more than one) formed in an annular pattern around the circumference of the wire member 2 at certain intervals in the lengthwise direction.

In the embodiment shown in Fig. 7, the hardened parts 4 are formed at equal intervals (pitches). However, they may be formed such that the intervals gradually increase or decrease in going from the base end to the forward end of the wire member 2. The hardened parts 4 formed in this way make the wire member (core wire) 2 gradually increase or decrease in flexural and torsional stiffness in going from the base end to the forward end.

In the embodiment shown in Fig. 7, the annular (stripy) hardened parts 4 have an equal width. However, they 4 may be formed such that their width gradually increases or decreases in going from the base end to the forward end of the wire member 2. The hardened parts 4 formed in this way make the wire member (core wire) 2 gradually increase or decrease in flexural and torsional stiffness in going from the base end to the forward end.

The hardened parts 41 shown in Fig. 7 may be formed in such a pattern that the adjoining annular parts are connected to each other (not shown) by circular, spiral, or reticulate parts.

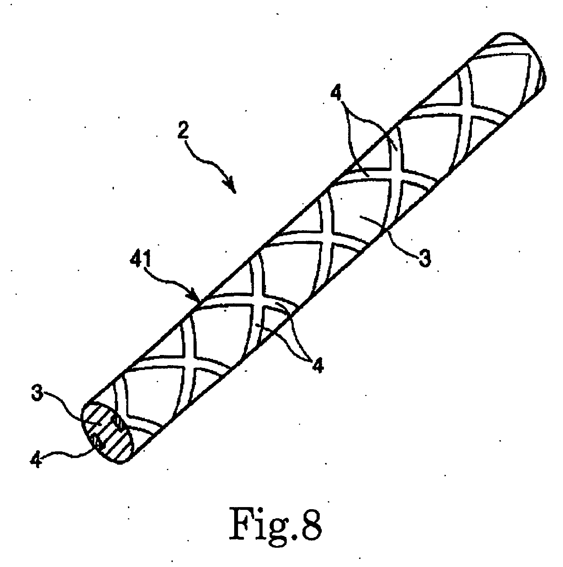

In the embodiment shown in Fig. 8, the wire member (core wire) 2 has the hardened parts 4 (in a reticulate pattern) formed on the periphery thereof. The pattern of the hardened parts 4 includes at leans two spirals running in mutually opposite directions.

The reticulate pattern of the hardened parts 4 may have a spiral pitch (or opening) which is constant or variable in the lengthwise direction of the wire member (core wire) 2 in the same way as mentioned above.

In the case where the hardened parts 4 in a reticulate pattern have a pitch which gradually deceases in going from the base end to the forward end, the wire member (core wire) 2 gradually decreases in flexural and torsional stiffness in going from the base end to the forward end.

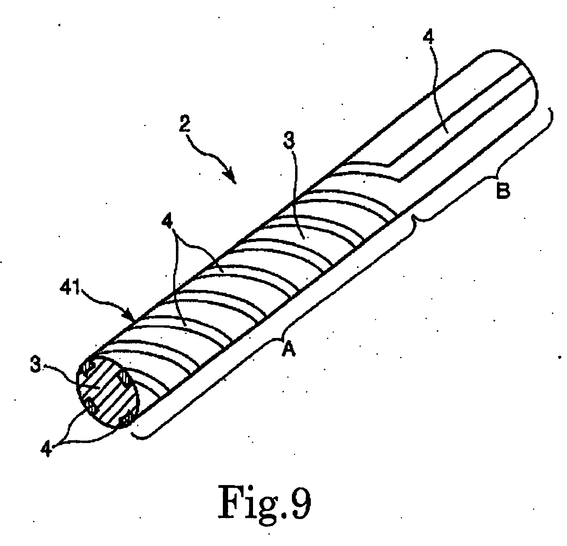

In the example shown in Fig. 9, the wire member (core wire) 2 has the hardened parts 4 (four in total) formed in the periphery thereof. Each of the hardened parts 4 includes a spiral (curved) part A and a straight part B, the latter being connected to the former and extending in the lengthwise direction of the wire member 2. The hardened parts 4 formed in this manner make the wire member (core wire) 2 increase in flexural and torsional stiffness in going from part B to part A.

The hardened parts 4 may be modified in configuration such that the number of straight parts B is larger than that of spiral parts A or the width of straight parts B is larger than that of spiral parts A. In this case, the wire member (core wire) 2 decreases in stiffness in going to the forward end.

In the example shown in Fig. 9, the spiral hardened parts 4 in part A may have a constant or varied pitch throughout its length. In the latter case, the pitch gradually increases (and hence the angle also gradually increases) in going to the vicinity of the straight part B. This configuration allows the wire member 2 to smoothly change in stiffness in going from part A to part B. The result is improved torque transmittability and kink resistance in the vicinity of the boundary between part A and part B.

The straight part B may be incorporated into the hardened part 4 shown in Figs. 2, 3, 4, 5, 7, and 8.

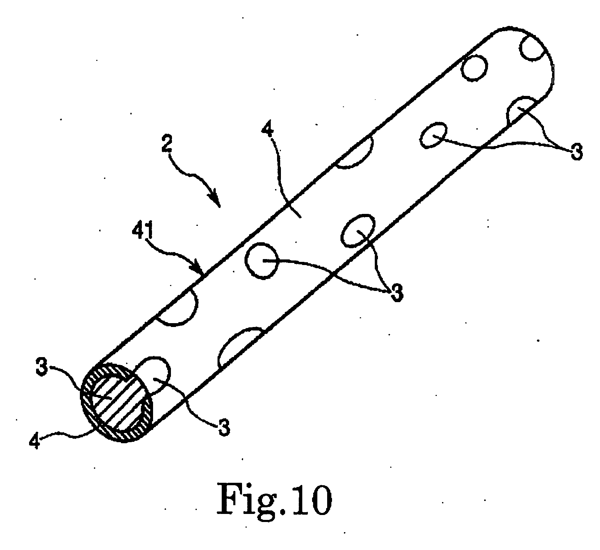

In the embodiment shown in Fig. 10, the wire member (core wire) 2 has the hardened part 4 formed in the entire periphery thereof, with the unhardened part 3 remaining sporadically as indicated by spots. The unhardened part 3 may be regularly arranged unlike the illustrated one.

The embodiment shown in Fig. 10 offers the advantage of imparting comparatively high stiffness to the wire member 2 because the hardened part 4 accounts for a larger portion than the unhardened part 3. Therefore, this structure should preferably be applied to the base end of the wire member 2.

The embodiment shown in Fig. 10 offers the advantage of imparting varied stiffness to the wire member 2 according as the density of the unhardened part 3 increases or decreases in the lengthwise direction of the wire member 2. For example, the unhardened part 3 may exist more densely in the forward end than in the base end. Alternatively, the thickness of the hardened part 4 may be varied so that the wire member 2 has stiffness varying from one position to another.

The unhardened part 3 in the periphery of the wire member 2 may take on any shape (such as rectangle and triangle) as well as circle or ellipse as shown in Fig. 10. It may also be uniform or varied in shape and size in the lengthwise direction of the wire member 2.

The wire member (core wire) 2 has the hardened part 4 (in layer form) covering the region [B], which is closer to the base end than the region [A], as shown in Fig. 11. The hardened part 4 in this form imparts higher stiffness to the wire member 2 than the odd-shaped part 41.

The hardened part 4 in the region [B] shown in Fig. 1 may have any shape which is not restricted to the one shown in Fig. 11. It may be straight as shown in Fig. 6 or mixed with the unhardened part 3 as shown in Fig. 10. The hardened part 4 in the region [B] may have a combination of the structure at its base end side as shown in Fig. 11 and the structure (straight) at its forward end side as shown in Fig. 6.

The hardened part 4 in the wire member 2 should have a cross section which has a specific shape and area as defined below with reference to Fig. 12 to 15.

The hardened part 4 formed in the wire member 2 has a thickness which varies in the widthwise direction as shown in Fig. 12. In other words, the depth is greatest at the center of the width and gradually decreases in going from the center to the edges.

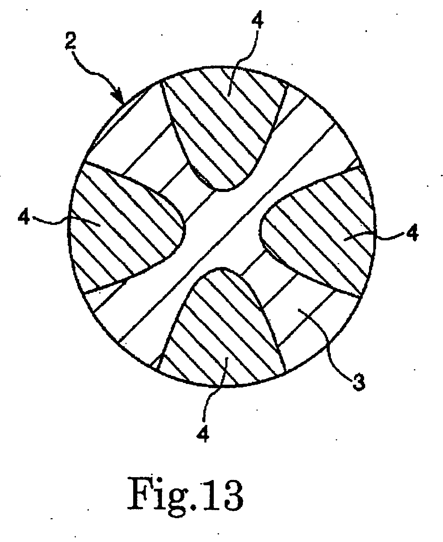

The hardened part 4 may be formed comparatively deep (even to the center) in the wire member 2 as shown in Fig. 13. The maximum depth of the hardened part 4 (or the position closest to the center of the wire member 2) may be uniform or varied for each of the hardened parts 4.

The maximum depth of the hardened part 4 may vary in the lengthwise direction of the wire member 2. For example, the maximum depth of the hardened part 4 may vary continuously or intermittently in going from the base end to the forward end of the wire member 2. In this case, the wire member 2 has gradually increasing flexibility and gradually decreasing stiffness in going from its base end to its forward end. This leads to the guide wire 1 having both improved steerability and safety.

In the case where the hardened part 4 is formed by irradiation with a laser beam and ensuing quenching, the maximum depth of the hardened part 4 may be attained by increasing the energy of the laser beam (which depends on the power of laser source, condensing density, temperature, and duration of irradiation).

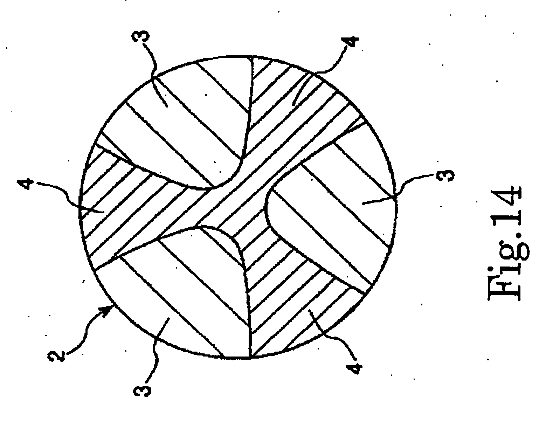

The hardened part 4 may be formed down to the center of the wire member 2 as shown in Fig. 14. In this case, the hardened parts 4 join together at the center of the wire member 2.

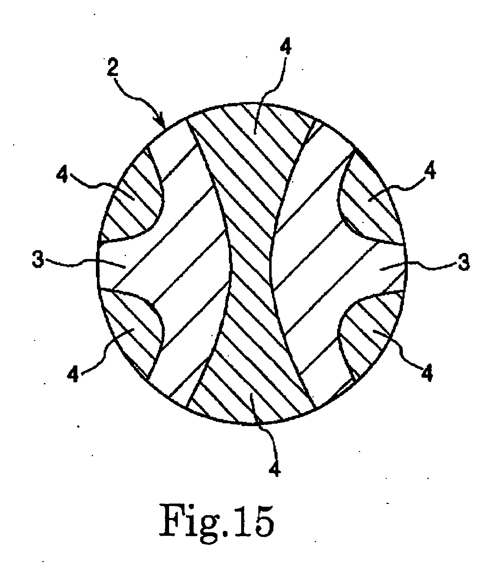

Also, the hardened part 4 may be formed as shown in Fig. 15. That is, some of the hardened parts 4 reach the center of the wire member 2 (joining together) and others do not.

The hardened part 4 and the unhardened part 3 in the wire member 2 may have any total sectional areas S₁ and So, respectively, with their ratio being 0 < S₁ < S₀ (condition 1). That part of the wire member 2 which satisfies this condition has comparatively high flexibility. Thus, the forward end of the wire member 2 should meet this condition. The condition 1 may be altered such that 0 < S₁ < 0.6 × S₀.

The hardened part 4 and the unhardened part 3 may have the total sectional areas S₁ and S₀, respectively, with their ratio being S₁ ≥ S₀ > 0 (condition 2). That part of the wire member 2 which satisfies this condition has comparatively high stiffness. Thus, the base end of the wire member 2 should meet this condition. The condition 2 may be altered such that 0.6 × S₁ ≥ S₀ > 0.

The wire member 2 may have the hardened part 4 which includes both the part that satisfies the condition 1 and the part that satisfies the condition 2. In other words, it may have the hardened part 4 which changes in its total sectional area S₁ in the lengthwise direction of the wire member (core wire) 2. For example, the hardened part 4 may have the total sectional area (S₁) which continuously or intermittently decreases in going from the base end to the forward end of the wire member 2. In this case, the wire member 2 gradually increases in flexibility and gradually decreases in stiffness in going from the base end to the forward end of the wire member 2. This leads to the guide wire 1 having both improved steerability and safety.

As mentioned above, the hardened part 4 may have uniform or partially varied hardness in the radial and/or lengthwise direction of the wire member 2. For example, hardness may be highest near the periphery of the wire member 2 and gradually decrease in going to the center of the wire member 2. In this case, the guide wire 1 has improved torque transmittability. In the case where the hardened part 4 is formed over the entire length from the base end to the forward end of the wire member 2, the hardened part 4 may vary in hardness from the base end to the forward end. In a typical example, the hardness is higher in the base end than in the forward end. In this case, the wire member 2 gradually increases in flexibility and gradually decreases in stiffness in going from the base end to the forward end of the wire member 2, in the same way as mentioned above.

The hardened part 4 may have a combination of two or more of the structures shown in Figs. 2 to 15.

According to the embodiments mentioned above, the hardened part 4 is formed in that portion of the wire member 2 which is approximately uniform in diameter. However, it may also be formed in that portion (15 or 16) of the wire member 2 which decreases in diameter in the lengthwise direction. The odd-shaped part 41 may also be formed in such portions. In this case, the decreasing diameter imparts flexibility to the wire member 2 while decreasing stiffness, and the hardened part 4 imparts stiffness to the wire member 2. This smoothly varying stiffness further improves the guide wire 1 in steerability and safety.

The wire member 2 has the coil 8 wound around the periphery of the forward end of the wire member 2. The coil 8 is a spirally wound member of thin wire. It covers at least the forward end of the wire member 2. (The forward end denotes the part whose diameter is smaller than that in the region A.) In the illustrated structure, the coil 8 is formed such that the forward end of the wire member 2 passes through the center thereof. Moreover, the inside of the coil 8 is not in contact with the forward end of the wire member 2.

The illustrated coil 8 is formed such that there is a gap between adjacent spiral thin wires in the absence of external force. This structure may be modified such that the thin wires are densely wound even in the absence of external force.

The coil 8 should preferably be formed from a metallic material, such as stainless steel, superelastic alloy, cobalt alloy, tungsten, and precious metals and alloy thereof (such as gold, platinum, and platinum-iridium alloy). Any material such as precious metals opaque to X-rays makes the guide wire 1 useful for contrastradiography when it is necessary to confirm the position of the forward end by X-ray illumination during insertion into the living body.

The coil 8 may be a combination of two or more coils. Moreover, it may be formed from different materials. For example, that side of the coil 8 which is close to the forward end may be formed from a material opaque to X-rays, and that side of the coil 8 which is close to the base end may be formed from a material (such as stainless steel) which is less opaque to X-rays. The overall length of the coil 8 is not specifically restricted; it should preferably be about 5 to 500 mm.

The coil 8 is fixed to the wire member 2 at its both ends with the fixing materials 11 and 12. The coil 8 is also fixed to the wire member 2 at its middle (close to the forward end) with the fixing material 13. The fixing materials 11, 12, and 13 are solder (brazing material) or adhesive. Fixing may be accomplished by welding instead of soldering. The edge of the fixing material 12 should be round so as not to damage the wall of the body cavity ,such as blood vessel.

The advantage of this embodiment is that the coil 8 covering the wire member 2 reduces the contact area, which leads to a reduced sliding resistance and hence to an improved steerability.

The coil 8 may have any shape in its section (such as square, rectangle, and ellipse) in place of circle as in this embodiment.

The wire member 2 has its surface covered entirely or partly with a plastic coating layer 9, as shown in Fig. 1. The plastic coating layer 9 serves various purposes. For example, it will reduce the friction (sliding resistance) of the guide wire 1 to improve steerability.

The plastic coating layer 9 to reduce the friction (sliding resistance) of the guide wire 1 should be formed from any of the following materials. The guide wire 1 with reduced friction moves and rotates easily in the catheter without bending, kinking, and twisting.

Those materials which reduce friction include, for example, polyolefins such as polyethylene and polypropylene, polyvinyl chloride, polyesters such as PET and PBT, polyamide, polyimide, polyurethane, polystyrene, polycarbonate, silicone resin, fluoroplastics (such as PTFE and ETFE), and their composite materials.

Preferable among these materials is a fluoroplastics (and its composite materials), which effectively reduces friction (sliding resistance) between the guide wire 1 and the inside wall of the catheter, thereby helping the guide wire 1 to move and/or rotate smoothly in the catheter without kinking, bending, and twisting which otherwise easily occur near the welded part.

In addition, fluoroplastics (or its composite material) can be applied for coating to the wire member 2 by baking or spraying in its molten state. The resulting plastics coating layer 9 has good adhesion to the wire member 2.

Silicone resin (or its composite material) of reaction curable type can be applied for coating to the wire member 2 at room temperature in a simple manner without heating. The resulting coating layer 9 firmly adheres to the wire member 2.

The plastics coating layer 9 may also serve to improve safety in operation of the guide 1 being inserted into the blood vessel. For this purpose, it should be formed from a flexible, elastic, or soft material.

Examples of the flexible materials include polyolefins such as polyethylene and polypropylene, polyvinyl chloride, polyester such as PET and PBT, polyamide, polyimide, polyurethane, polystyrene, silicone resin, thermoplastic elastomer such as polyurethane elastomer, polyester elastomer, and polyamide elastomer, rubbers such as latex rubber and silicone rubber, and their composite materials.

The resin coating layer 9 formed from a rubbery material such as thermoplastic elastomer and rubbers makes the forward end of the guide wire 1 flexible, thereby eliminating the possibility of it damaging the wall of the blood vessel at the time of insertion. The resin coating layer 9 may have a laminate structure composed of two or more layers.

The resin coating layer 9 may have any thickness without specific restrictions, which may be properly selected according to its object, constituent material, and forming method. Its preferred thickness is about 1 to 100 µm*,* particularly about 1 to 30 µm on average. It does not produce its effect and it is liable to peeling if it is excessively thin. It physically affects the wire member 2 and it is liable to peeling if it is excessively thick.

The resin coating layer 9 firmly adheres to the wire member (core wire) 2 formed from the above-mentioned material, and it more firmly adheres to the surface of the hardened part 4. Incidentally, for better adhesion, an intermediate layer may be interposed between the resin coating layer 9 and the surface of the wire member 2.

The guide wire 1 should preferably have a coating layer of hydrophilic material on at least the surface of its forward end. The guide wire 1 according to this embodiment has a coating layer of hydrophilic material on the surface of the region extending from its forward end to the base of the tapering part 16. The coating layer improves the slidability and steerability of the guide wire 1 as the hydrophilic material swells to produce the lubricating effect.

Examples of the hydrophilic material include cellulosic polymer, polyethylene oxide polymer, maleic anhydride polymer such as methyl vinyl ether-maleic anhydride copolymer, acrylamide polymer such as polyacrylamide and polyglycidylmethacrylate-dimethylacrylamide (PGM-DMAA) block copolymer, water-soluble nylon, polyvinyl alcohol, and polyvinylpyrrolydone.

The above-mentioned hydrophilic material produces its lubricating effect upon water absorption, thereby reducing friction (sliding resistance) between the guide wire 1 and the inside wall of the catheter used in combination therewith. The reduced friction improves the steerability of the guide wire 1.

The guide wire 1 constructed as mentioned above partially varies in stiffness owing to the hardened part 4 formed in the wire member 2. That is, it may have comparatively high stiffness at its base end and adequate flexibility at its forward end. The combination of stiffness and flexibility contributes to good pushability, torque transmittability, steerability, and safety. This effect is produced even in the case of a thin guide wire.

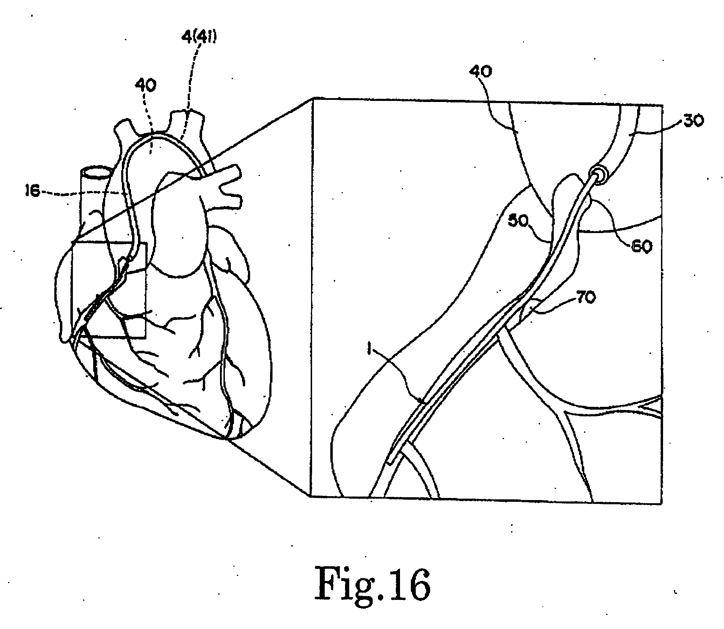

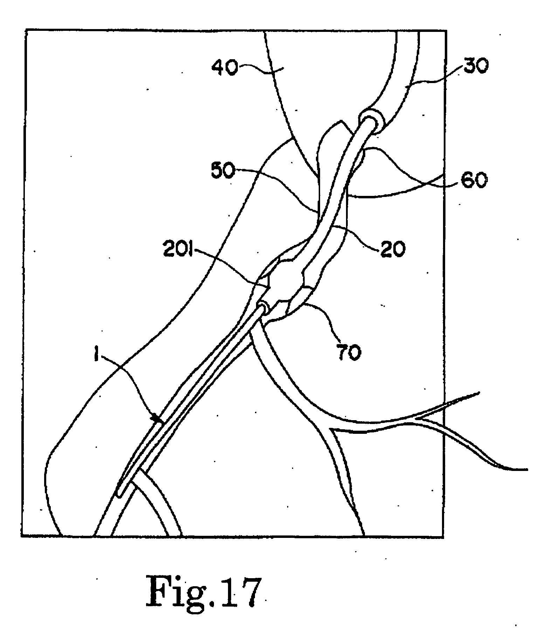

Figs. 16 and 17 illustrate how the guide wire 1 of the present invention is used for PTCA.

Reference numerals in Figs. 16 and 17 are defined as follows.
40 ... aortic arch
50 ... right coronary artery
60 ... opening of right coronary artery
70 ... narrow segment (lesion) of vessel
30 ... guiding catheter to guide with certainty the guide wire 1 from the femoral artery to the right coronary artery
20 ... balloon catheter to expand the narrow segment which has the expandable/collapsible balloon 201 at its forward end

The procedure mentioned below is carried out with the help of X-ray radioscopy.

The first step starts with insertion of the guide wire 1 with its forward end projecting from the guiding catheter 30 from the opening 60 of the right coronary artery into the right coronary artery 50, as shown in Fig. 16. The guide wire 1 is advanced through the right coronary artery 50 until its forward end reaches the position beyond the narrow segment 70. The guide wire 1 placed at this position ensures the passage of the balloon catheter 20. At this time, the tapering part 16 of the guide wire 1 resides in that part of the aortic arch which is close to the descending aorta. The hardened part 4 extends throughout the aortic arch 40.

In the next step shown in Fig. 17, the balloon catheter 20, which has been slipped over the guide wire 1 from its base end, is advanced so that its forward end projects from the forward end of the guiding catheter 30, and the balloon catheter 20 is advanced further along the guide wire 1 and inserted into the right coronary artery 50 from its opening 60 until the balloon 201 reaches the narrow segment 70 of the vessel.

In the third step, the balloon 201 is expanded by injection of a balloon expanding fluid from the base end of the balloon catheter 20, so that the narrow segment 70 is expanded. The expanded balloon physically expands deposit such as cholesterol on the narrow segment 70 of the vessel, thereby restoring the blood flow.

The guide wire according to the above-mentioned embodiment may be modified by altering its structure or by adding any constituents within the scope of the present invention.

The guide wire according to the present invention may be used not only for PTCA but also for therapy of CTO chromic total occlusion (CTO), angiography, and endoscopic operation.

### [Examples]

A detailed description is given below of the example of the present invention.

### Example 1

A wire member specified below was prepared.

Core wire : 0.34 mm in diameter, 1.5 m long, made of stainless steel (SUS302) composed of 0.12 wt% C, 0.39 wt% Si, 0.80 wt% Mn, 0.029 wt% P, 0.001 wt% S, 8.51 wt% Ni, 18.82 wt% Cr, and Fe (remainder).

The core wire had its surface irradiated with a YAG laser beam with an output of 500 W by using a YAG laser irradiating apparatus made by ROFIN-SINAR Technologies Inc. During irradiation with a laser beam the core was placed in an argon gas stream. After irradiation, the core wire was quenched to normal temperature within about two seconds in an argon gas stream. The irradiation and ensuing quenching gave a stripe hardened part (0.1 mm wide over the entire length of the core wire) with a pitch of 5 mm. The hardened part had an average thickness of 0.1 mm.

The core wire was tested for Vickers hardness (Hv) at several positions. The measured values were 660 to 700 on the surface of the core wire (or the surface of the unhardened part) and 720 to 770 on the surface of the hardened part.

### Comparative Example

The same stainless steel wire as in Example 1 was used as the wire member (core wire). Hardening was not performed on the surface of the core wire.

### <Torque test>

The samples of the wire member prepared in Example 1 and Comparative Example were tested for torque transmittability.

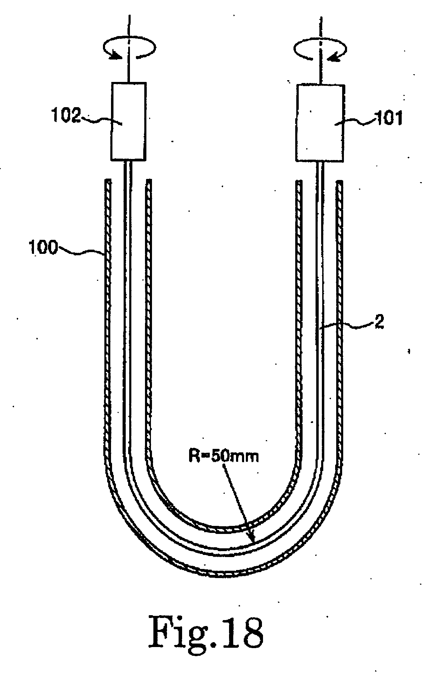

The sample of the wire member was inserted into a U-shaped plastic tube 100, having an inside diameter of 2 mm and a radius of curvature of 50 mm (at the center of the tube), with its both ends projecting from the ends of the plastic tube 100, as shown in Fig. 18.

One end of the wire member was provided with a device 101 to rotate the wire, and the other end of the wire member was provided with a device 102 to measure the angle of rotation.

The device 101 was turned in one direction at 3 rpm, with the plastic tube 100 fixed. The wire member transmits the rotating force from one end to the other, causing the device 102 to turn in the opposite direction. In this way the angle of rotation was measured by the device 102.

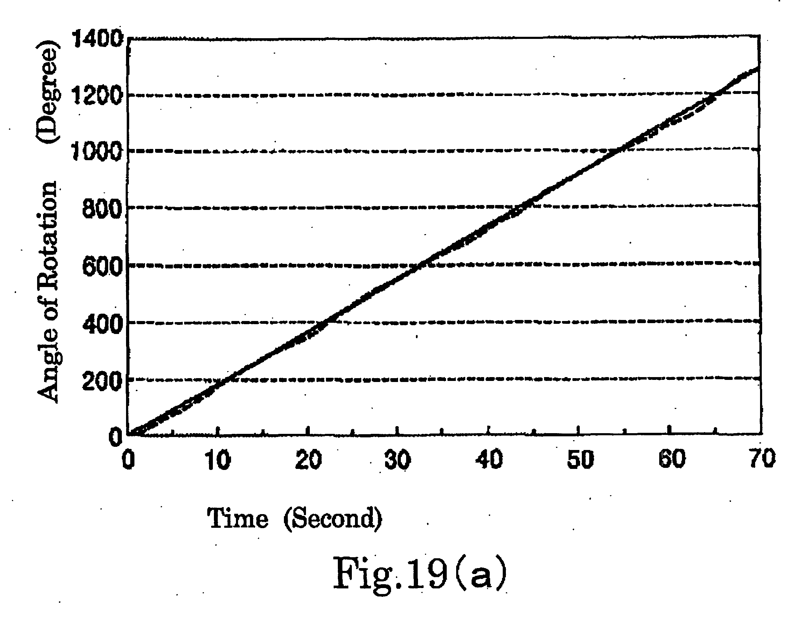

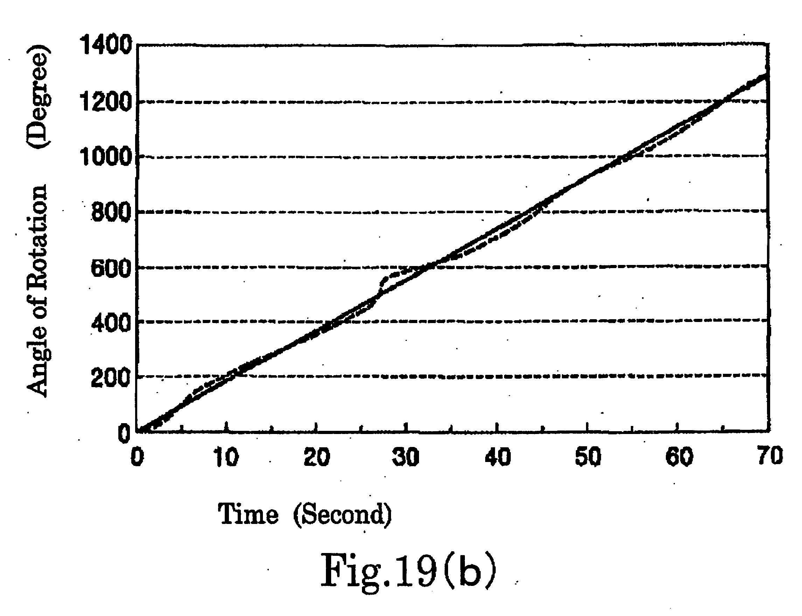

The angle of rotation produced by the device 101 and the angle of rotation measured by the device 102 were plotted against time for the samples in Example 1 and Comparative Example as shown in Figs. 19A and 19B, respectively. Solid lines denote the input angle and broken lines denote the output angle. The more the two lines are close to each other, the better the wire member is in torque transmittability. The more the two lines are separate from each other, the worse the wire member is in torque transmittability.

It is noted from Fig. 19A that the wire member 1 in Example 1, which has the hardened part, allowed the input angle (denoted by a solid line) and the output angle (denoted by a broken line) to change almost in the same way over the entire period of measurements. This suggests good torque transmittability. By contrast, it is noted from Fig. 19B that the wire member 1 in Comparative Example, which does not have the hardened part, allowed the input angle and the output angle to change differently, particularly after 30 seconds and 40 seconds. This suggests poor torque transmittability.

### Example 2

The same procedure as in Example 1 is repeated to prepare the wire member except that the hardened part in the periphery of the wire member was changed in pattern as shown in Fig. 2. The pattern consists of three spiral stripes, with an average width of 0.05 mm and a pitch of 5 mm over the entire length. The hardened part is 0.05 mm deep on average.

### Example 3

The same procedure as in Example 2 is repeated to prepare the wire member except that the hardened part in the periphery of the wire member was changed in depth and width (0.08 mm on average), so that the three hardened parts join together at the center of the wire member, as shown in Fig. 14.

### Example 4

The same procedure as in Example 1 is repeated to prepare the wire member except that the hardened part in the periphery of the wire member is changed in pattern as shown in Fig. 6. The pattern includes three straight parallel stripes equally apart, with an average width of 0.05 mm over the entire length. The hardened part is 0.05 mm deep on average.

### Example 5

The same procedure as in Example 4 is repeated to prepare the wire member except that the hardened part in the periphery of the wire member is changed in depth and width (0.1 mm on average), so that the three hardened parts join together at the center of the wire member, as shown in Fig. 14.

### Example 6

The same procedure as in Example 1 is repeated to prepare the wire member except that the hardened part in the periphery of the wire member is changed in pattern as shown in Fig. 8. The pattern includes reticulate stripes, with a width of 0.05 mm over the entire length.

### Example 7

The same procedure as in Example 1 is repeated to prepare the wire member except that the hardened part in the periphery of the wire member is changed in pattern as shown in Fig. 10. The hardened part covers the entire surface excluding the unhardened parts which look like a polka-dotted pattern.

The samples in Examples 2 to 7 are tested for torque transmittability in the same way as mentioned above.

Disclosed herein is a guide wire which, even with its diameter reduced, has adequate stiffness at specific positions to ensure good steerability and kink resistance. It is composed of the wire member which is a metal core wire and the coil covering the forward end thereof. The core wire has in the periphery thereof the hardened part and the unhardened part which are adequately patterned. The hardened part may have the odd-shaped part, which includes three stripes spirally arranged at equal intervals in the circumferential direction. The hardened part is preferably formed by irradiation with a laser beam and ensuing quenching.

## Claims

1. A guide wire (1) comprises a wire member (2) having a flexible core wire and a hardened part (4) formed at least on the surface thereof by hardening treatment, wherein the hardened part (4) has a specific pattern (41) in a specific region (A) along the lengthwise direction of the core wire, the specific pattern (41) assuming a straight pattern, curved pattern, annular pattern, spiral pattern, or reticulate pattern, or a combination of two or more of the patterns, wherein the core wire is formed from a ferroalloy, the ferroalloy being at least one species selected from the group including stainless steel, piano wire, iron-cobalt alloy, any other cobalt alloys, carbon steel, mild steel, hard steel, nickel steel, nickel-chrome steel, and nickel-chrome-molybdenum steel,
the wire member further having an unhardened part at least on the surface, the unhardened part having a metallographic structure of austenite and the hardened part having one or more of a structure of martensite, pearlite, and bainite
**characterized in that**
the guide wire (1) has a part in which the hardened parts (4) in the specific pattern (41) are formed at intervals decreasing in going toward one end of the core wire.

2. The guide wire (1) as defined in Claim 1, wherein the hardened part (4) has an average Vickers hardness of 1.02 K to 3.5 K, where K denotes a Vickers hardness at that part (3) of the core wire which has not yet undergone hardening treatment.

3. The guide wire (1) as defined in any one of the preceding Claims, which has a part in which the hardened part (4) in the periphery of the core wire assumes a stripe pattern changing in width along the lengthwise direction of the core wire.

4. The guide wire (1) as defined in any one of the preceding Claims, which has the hardened part (4) such that at least part of it is near or at the center of the core wire.

5. The guide wire (1) as defined in any one of the preceding Claims, which has a part in which the total sectional area of the hardened part (4) is smaller than the total sectional area of the unhardened part (3) without hardening treatment of the core wire.

6. The guide wire (1) as defined in any one of the preceding Claims, which has a part in which the total sectional area of the hardened part (4) is equal to or larger than the total sectional area of the unhardened part (3) without hardening treatment of the core wire.

7. The guide wire (1) as defined in any one of the preceding Claims, which has a part in which the total sectional area of the hardened part (4) varies in the lengthwise direction of the core wire.

8. The guide wire (1) as defined in any one of the preceding Claims, wherein the core wire is made of a metallic material containing no less than 0.1 wt% of carbon.

9. The guide wire (1) as defined in any one of the preceding Claims, wherein the hardened part (4) has a part varying in hardness in the radial direction and/or the lengthwise direction of the wire member (2).

10. The guide wire (1) as defined in any one of the preceding Claims, wherein the hardened part (4) is formed by heating and quenching.

11. The guide wire (1) as defined in Claim 11, wherein the heating is accomplished by irradiation with a laser beam.

12. The guide wire (1) as defined in any one of the preceding Claims, wherein the hardened part (4) is formed next to the specific pattern (41) and has an approximately straight part arranged in the lengthwise direction of the core wire.

13. The guide wire (1) as defined in any one of the preceding Claims, wherein the hardened part (4) is arranged in the specific pattern (41) more densely in the base end side of the wire member (2) than in the forward end side of the wire member (2).

14. The guide wire (1) as defined in any one of the preceding Claims, which has a covering layer (9) that covers the wire member (2).

15. The guide wire as defined in any one of the preceding Claims, wherein the wire member is formed of a eutectoid carbon steel or a nickel-chrome-molybdenum steel.

## Patentansprüche

1. Ein Führungsdraht (1) umfasst ein Drahtelement (2), das einen flexiblen Kerndraht und ein gehärtetes Teil (4) aufweist, das zumindest an dessen Oberfläche durch eine Härtungsbehandlung ausgebildet ist, wobei das gehärtete Teil (4) ein bestimmtes Muster (41) in einem bestimmten Bereich (A) entlang der Längsrichtung des Kerndrahts aufweist, das bestimmte Muster (41) ein gerades Muster, ein gekrümmtes Muster, ein ringförmiges Muster, ein spiraliges Muster oder ein verästeltes Muster oder eine Kombination aus zwei oder mehr der Muster annimmt, wobei der Kerndraht aus einer Eisenlegierung ausgebildet ist, die Eisenlegierung zumindest eine aus einer Gattung ist die aus der Gruppe mit rostfreiem Stahl, Klavierdraht, einer Eisen-Kobalt-Legierung, einer anderen Kobaltlegierung, Kohlenstoffstahl, Weichstahl, gehärtetem Stahl, Nickel-Stahl, Nickel-Chrom-Stahl und Nickel-Chrom-Molybdän-Stahl ausgebildet ist,
wobei das Drahtelement außerdem ein ungehärtetes Teil an zumindest der Oberfläche aufweist, und das ungehärtete Teil eine metallographische Struktur aus Austenit aufweist, und das gehärtete Teil eine oder mehr einer Struktur aus Martensit, Perlit und Bainit aufweist,
**dadurch gekennzeichnet, dass**
der Führungsdraht (1) ein Teil aufweist, in dem die gehärteten Teile (4) in dem bestimmten Muster (41) in Abständen ausgebildet sind, die sich zu einem Ende des Kerndrahts hin verringern.

2. Führungsdraht (1) wie in Anspruch 1 definiert, wobei das gehärtete Teil (4) eine durchschnittliche Vickershärte von 1,02 K bis 3,5 K aufweist, wobei K eine Vickershärte an dem Teil (3) des Kerndrahts bezeichnet, der gerade nicht einer Härtungsbehandlung unterzogen wurde.

3. Führungsdraht (1) wie in einem der vorangehenden Ansprüche definiert, der ein Teil aufweist, in dem das gehärtete Teil (4) in dem Umfang des Kerndrahts ein Streifenmuster annimmt, dessen Breite sich entlang der Längsrichtung des Kerndrahts ändert.

4. Führungsdraht (1) wie in einem der vorangehenden Ansprüche definiert, der das gehärtete Teil (4) derart aufweist, dass zumindest ein Teil davon nahe oder an der Mitte des Kerndrahts liegt.

5. Führungsdraht (1) wie in einem der vorangehenden Ansprüche definiert, der ein Teil aufweist, in dem die gesamte Schnittfläche des gehärteten Teils (4) kleiner als die gesamte Schnittfläche des ungehärteten Teils (3) ohne Härtungsbehandlung des Kerndrahtes ist.

6. Führungsdraht (1) wie in einem der vorangehenden Ansprüche definiert, der ein Teil aufweist, in dem die gesamte Schnittfläche des gehärteten Teils (4) gleich oder größer als die gesamte Schnittfläche des ungehärteten Teils (3) ohne Härtungsbehandlung des Kerndrahts ist.

7. Führungsdraht (1) wie in einem der vorangehenden Ansprüche definiert, der ein Teil aufweist, in dem die gesamte Schnittfläche des gehärteten Teils (4) in der Längsrichtung des Kerndrahts variiert.

8. Führungsdraht (1) wie in einem der vorangehenden Ansprüche definiert, wobei der Kerndraht aus einem metallischen Material hergestellt ist, das nicht weniger als 0,1 Gewichtsprozent Kohlenstoff enthält.

9. Führungsdraht (1) wie in einem der vorangehenden Ansprüche definiert, wobei das gehärtete Teil (4) ein Teil aufweist, dessen Härte in der radialen Richtung und/oder der Längsrichtung des Drahtelements (2) variiert.

10. Führungsdraht (1) wie in einem der vorangehenden Ansprüche definiert, wobei das gehärtete Teil (4) durch Erwärmen und Abkühlen ausgebildet ist.

11. Führungsdraht (1) wie in Anspruch 11 definiert, wobei das Wärmen durch Bestrahlung mit einem Laserstrahl herbeigeführt wird.

12. Führungsdraht (1) wie in einem der vorangehenden Ansprüche definiert, wobei das gehärtete Teil (4) als nächstes an dem bestimmten Muster (41) ausgebildet ist und ein annähernd gerades Teil in Längsrichtung des Kerndrahts angeordnet aufweist.

13. Führungsdraht (1) wie in einem der vorangehenden Ansprüche definiert, wobei das gehärtete Teil (4) in dem bestimmten Muster (41) an dem Basisende des Drahtelements (2) dichter als an der vorderen Endseite des Drahtelements (2) angeordnet ist.

14. Führungsdraht (1) wie in einem der vorangehenden Ansprüche definiert, die eine Abdeckschicht (9) aufweist, die das Drahtelement (2) abdeckt.

15. Führungsdraht wie in einem der vorangehenden Ansprüche definiert, wobei das Drahtelement aus einem eutektischen Kohlenstoffstahl oder einem Nickel-Chrom-Molybdänstahl ausgebildet ist.

## Revendications

1. Fil guide (1) comprenant un élément de fil (2) ayant une tige d'armature souple et une partie durcie (4) formée au moins sur sa surface par traitement de durcissement, où la partie durcie (4) a un motif spécifique (41) dans une région spécifique (A) le long de la direction de la longueur de la tige d'armature, le motif spécifique (41) adoptant un motif linéaire, un motif courbé, un motif annulaire, un motif spiral, ou un motif réticulé, ou une combinaison de deux des motifs ou plus, où la tige d'armature est réalisée en ferro-alliage, le ferro-alliage étant au moins une espèce sélectionnée du groupe comportant l'acier inoxydable, le fil de piano, l'alliage fer-cobalt, tout autre alliage de cobalt, l'acier au carbone, l'acier doux, l'acier dur, l'acier au nickel, l'acier au nickel-chrome, et l'acier au nickel-chrome molybdène,
l'élément de fil ayant en outre une partie non-durcie au moins sur la surface, la partie non-durcie ayant une structure métallographique d'austénite et la partie durcie ayant une ou plusieurs structures de martensite, de perlite, et de bainite
**caractérisé en ce que** le fil guide (1) a une partie dans laquelle les parties durcies (4) dans le motif spécifique (41) sont formées à des intervalles qui se réduisent en allant vers une extrémité de la tige d'armature.

2. Fil guide (1) tel que défini dans la revendication 1, dans lequel la partie durcie (4) a une dureté Vickers moyenne de 1,02 K à 3,5 K, où K indique une dureté Vickers au niveau de cette partie (3) de la tige d'armature qui n'a pas encore subi de traitement de durcissement.

3. Fil guide (1) tel que défini dans l'une quelconque des revendications précédentes, qui a une partie dans laquelle la partie durcie (4) dans la périphérie de la tige d'armature adopte un motif de bande changeant en largeur le long de la direction de la longueur de la tige d'armature.

4. Fil guide (1) tel que défini dans l'une quelconque des revendications précédentes, qui a la partie durcie (4) de sorte qu'au moins une partie de celle-ci soit proche ou au centre de la tige d'armature.

5. Fil guide (1) tel que défini dans l'une quelconque des revendications précédentes, qui a une partie dans laquelle la surface totale en coupe de la partie durcie (4) est inférieure à la surface totale en coupe de la partie non-durcie (3) sans traitement de durcissement de la tige d'armature.

6. Fil guide (1) tel que défini dans l'une quelconque des revendications précédentes, qui a une partie dans laquelle la surface totale en coupe de la partie durcie (4) est supérieure ou égale à la surface totale en coupe de la partie non-durcie (3) sans traitement de durcissement de la tige d'armature.

7. Fil guide (1) tel que défini dans l'une quelconque des revendications précédentes, qui a une partie dans laquelle la surface totale en coupe de la partie durcie (4) varie dans la direction de la longueur de la tige d'armature.

8. Fil guide (1) tel que défini dans l'une quelconque des revendications précédentes, dans lequel la tige d'armature est réalisée en matériau métallique ne contenant pas moins de 0,1% en poids de carbone.

9. Fil guide (1) tel que défini dans l'une quelconque des revendications précédentes, dans lequel la partie durcie (4) a une partie de dureté variable dans la direction radiale et/ou la direction de la longueur de l'élément de fil (2).

10. Fil guide (1) tel que défini dans l'une quelconque des revendications précédentes, dans lequel la partie durcie (4) est formée par chauffage et refroidissement.

11. Fil guide (1) tel que défini dans la revendication 11, dans lequel le chauffage est réalisé par irradiation avec un faisceau laser.

12. Fil guide (1) tel que défini dans l'une quelconque des revendications précédentes, dans lequel la partie durcie (4) est formée à côté du motif spécifique (41) et a une partie approximativement rectiligne agencée dans la direction de la longueur de la tige d'armature.

13. Fil guide (1) tel que défini dans l'une quelconque des revendications précédentes, dans lequel la partie durcie (4) est agencée dans le motif spécifique (41) de manière plus dense du côté extrémité de base de l'élément de fil (2) que du côté extrémité avant de l'élément de fil (2).

14. Fil guide (1) tel que défini dans l'une quelconque des revendications précédentes, qui a une couche de revêtement (9) qui couvre l'élément de fil (2).

15. Fil guide tel que défini dans l'une quelconque des revendications précédentes, dans lequel l'élément de fil est réalisé en acier eutectoïde au carbone ou en un acier au nickel-chrome-molybdène.
